# EUROPEAN PATENT APPLICATION

(11) **EP 2 327 383 A1**
(43) Veröffentlichungstag der Anmeldung: **01.06.2011**
(21) Anmeldenummer: 09806906.5
(22) Anmeldetag: 12.03.2009
(51) Int. Cl.: A61F 9/008, A61N 5/067

(54) **VERFAHREN ZUR BEHANDLUNG VON KERTATOKONUS MITTELS UV-STRAHLUNG UND VORRICHTUNG ZUR AUSFÜHRUNG DIESES VERFAHRENS**

(30) Priorität: 14.08.2008 RU 2008133298
(71) Anmelder: Anisimov, Sergey Igorevich, Moscow 105-318 (RU)
(72) Erfinder: Anisimov, Sergey Igorevich, Moscow 105-318 (RU)
(74) Vertreter: Kietzmann, Manfred
(86) Internationale Anmeldenummer: PCT/RU2009/000121
(87) Internationale Veröffentlichungsnummer: WO 2010/019073

(57) **Zusammenfassung**

Die Erfindung bezieht sich auf die Ophthalmologie und wird zur Behandlung des Keratokonus eingesetzt. In der Hornhaut werden eine oder mehrere unterschiedlich geformte UV-Bestrahlungsfelder gebildet, u. a. in Form von konzentrischen Kreisen, Bögen, parallelen Linien, Kästchen, eines Gitters, von Spiralen bzw. anderen geometrischen Figuren. Eine weitere Variante besteht darin, dass das UV-Licht polarisiert und unter Ausnutzung des Polarisationseffektes des Lichts durch die Hornhaut und durch Ausrichtung der Polarisationsebene des UV-Lichts zur Polarisationsebene des Lichts durch die Hornhaut unter einem Winkel von 1 bis 180 Grad die Einwirkungstiefe des UV-Lichts reguliert wird. Die Vorrichtung zur Behandlung des Keratokonus besteht aus Fokussierelementen, die auf einer optischen Achse mit der UV-Lichtquelle liegen, und einem Diaphragma. Das Diaphragma ist auf einer optischen Achse mit der UV-Lichtquelle angeordnet und als Maske mit einander abwechselnden lichtdurchlässigen und lichtundurchlässigen Abschnitten, die die Form von konzentrischen Kreisen, Bögen, parallelen Linien, Kästchen, eines Gitters, von Spiralen bzw. anderer geometrischer Figuren haben, ausgeführt. In einer weiteren Variante besteht die Vorrichtung aus optischen Fokussierelementen, die auf einer optischen Achse mit der UV-Lichtquelle liegen, einem Diaphragma und einem Polarisator.

## Beschreibung

### I. Anwendungsgebiet

Die Erfindung bezieht sich auf die Medizin und betrifft konkret die Augenheilkunde. Sie kann zur Behandlung des Keratokonus mit UV-Strahlen eingesetzt werden.

### II. Zugrunde liegender Stand der Technik

Das Verfahren und die Vorrichtung zur Behandlung des Keratokonus mit UV-Strahlen, die mit voller Apertur auf die Hornhaut wirken, sind bekannt (Wollensak G., Spörl E., Seiler T. Treatment of keraoconus by Kollagen cross linking. Der Ophtalmologe: Zeitschrift der Deutschen Ophthalmologischen Gesellschaft, 2003, Jan., 100(1): S. 44-49).

Dieses Verfahren schädigt jedoch wegen der Nebenwirkung der UV-Strahlen die Hornhaut.

Bei der UV-Bestrahlung der Hornhaut erfolgt eine Photopolymerisation der Kollagen-Moleküle. Die Photopolymerisation wird durch nicht toxische, wasserlösliche Photomediatoren aktiviert. Dadurch, dass diese die Strahlen in der Tiefe absorbieren, soll das tiefere Hornhautgewebe, insbesondere die endothelialen Zellen, vor Schädigungen geschützt werden. Als ein solcher Photomediator kann Riboflavin dienen, insbesondere dessen 1%-ige Lösung. Die Vernetzung des Kollagens wird als Crosslinking bezeichnet und kann auf unterschiedlichen Ebenen der komplizierten Kollagen-Struktur erfolgen. Beim Crosslinking entstehen zusätzliche Verstrebungen im Tropokollagen, das die Primärstruktur dieses Eiweißes formt, und es bilden sich zusätzliche Verstrebungen zwischen den primären Kollagenstrukturen. Und schließlich können neue Brücken zwischen den Mikrofibrillen des Kollagens entstehen. All dies führt zu einer verstärkten Aggregation des Hornhautkollagens, seiner Härtung und Festigung. Die klinische Wirkung entsteht durch diese Veränderungen der biomechanischen Parameter der Hornhaut, die es ermöglichen, die Hornhautkrümmung bei Keratokonus zu stabilisieren.

Die Bestrahlung der Hornhaut aus einer UV-Lichtquelle mit voller Apertur, die eine gleichmäßige Belichtung der gesamten Hornhautfläche gewährleistet, kann jedoch eine Verbrennung durch das UV-Licht nach sich ziehen und die ungenügende Stärke der Hornhaut, die geringer ist als die Einwirkungstiefe der UV-Strahlen, kann zu einer Schädigung der endothelialen Zellen und Ausbildung von Komplikationen in Form einer Hornhautdystrophie führen. In einer Reihe von Fällen mussten spezielle Riboflavin-Mischungen mit Dextran verwendet werden, mit denen die Hornhaut durch ein zeitweiliges Ödem verdickt werden konnte. Das verringert natürlich die Wirkung des Crosslinkings des Hornhautkollagens bei Keratokonus an sich.

### III. Offenbarung der Erfindung

Das technische Ergebnis, das bei der Umsetzung der Erfindung erreicht werden soll, besteht in der Reduzierung der Nebenwirkungen.

Das genannte technische Ergebnis wird dadurch erreicht, dass das Verfahren zur Behandlung des Keratokonus folgendermaßen umgesetzt wird. Eine Lokalanästhesie wird vorgenommen und in das Auge ein Photomediator geträufelt - eine 1%-ige Riboflavin-Lösung 30 Minuten lang alle zwei Minuten je einen Tropfen. Die Hornhaut wird mit UV-Licht von höchstens 5 mW/cm² zwischen 1 und 90 Minuten bestrahlt, indem dabei eine oder mehrere Bestrahlungsfelder in Form von konzentrischen Kreisen, eines Gitters, von Kästchen, Spiralen, Bögen, parallelen Linien oder anderen geometrischen Figuren gebildet werden. Damit entsteht ein originelles Gebilde, es wird die erforderliche Festigkeit der Hornhaut bei geringer Einwirkungsfläche erreicht, was es ermöglicht, bei gleichbleibender Behandlungseffektivität die Wahrscheinlichkeit von Nebenwirkungen zu verringern bzw. die Nebenwirkungen zu mildern und die Herausbildung einer oder mehrerer Bestrahlungsfelder in Form von konzentrischen Kreisen, eines Gitters, von Kästchen, Spiralen, Bögen, parallelen Linien oder anderen geometrischen Figuren zu gewährleisten.

Das genannte technische Ergebnis wird auch dadurch erzielt, dass das Behandlungsverfahren folgendermaßen realisiert wird. Es erfolgt eine Lokalanästhesie. In das Auge wird der Photomediator geträufelt - eine 1%-ige Riboflavin-Lösung 30 Minuten lang alle zwei Minuten je einen Tropfen. Das UV-Licht wird polarisiert, die Polarisationsebene unter einem Winkel von 1 bis 180 Grad zur Polarisationsebene der Hornhaut ausgerichtet. Die Hornhaut wird mit UV-Licht von höchstens 5 mW/cm² zwischen 1 und 90 Minuten bestrahlt. Die Polarisation des UV-Lichts und die Ausrichtung seiner Polarisationsebene zur Polarisationsebene der Hornhaut unter einem Winkel von 1 bis 180 Grad ermöglichen es, durch den Lichtpolarisationseffekt durch die Hornhaut die Einwirkungstiefe der UV-Strahlen zu regulieren, indem die UV-Wirkung auf die gegenüber einer Verbrennung durch Licht empfindlichen endothelialen Zellen verringert wird, was wiederum bei gleichbleibender Behandlungseffektivität die Wahrscheinlichkeit von Nebenwirkungen des UV-Lichts auf die Hornhaut verringert bzw. mögliche Nebenwirkungen mildert.

Das technische Ergebnis wird auch dadurch erzielt, dass die Vorrichtung zur Behandlung des Keratokonus ein Gehäuse mit innen liegender UV-Lichtquelle, ein damit verbundenes Netz- und Bedienungsgerät, optische Fokussierelemente, die auf einer optischen Achse mit der UV-Lichtquelle liegen, und ein Diaphragma einschließt.

Das Diaphragma ist als Maske mit einander abwechselnden lichtdurchlässigen und lichtundurchlässigen Abschnitten ausgeführt, die die Form von konzentrischen Kreisen, eines Gitters, von Kästchen, Spiralen, Bögen, parallelen Linien oder anderen geometrischen Figuren haben. Das Diaphragma soll auf einer optischen Achse mit der UV-Lichtquelle und den optischen Fokussierelementen angeordnet werden.

Das Diaphragma kann im Gehäuse der Vorrichtung oder außerhalb angeordnet sein.

In einer anderen Variante wird das technische Ergebnis dadurch erreicht, dass die Vorrichtung zur Behandlung des Keratokonus ein Gehäuse mit darin liegender UV-Lichtquelle, ein damit verbundenes Netz- und Bedienungsgerät, optische Fokussierelemente, die auf einer optischen Achse mit der UV-Lichtquelle liegen, ein Diaphragma und einen Polarisator einschließt, der auf einer optischen Achse mit der UV-Lichtquelle und den optischen Fokussierelementen angeordnet und so ausgeführt sein soll, dass die Polarisationsebene des UV-Lichts zur Polarisationsebene der Hornhaut im Bereich 1 - 180 Grad ausgerichtet werden kann.

Der Polarisator kann im Vorrichtungsgehäuse oder außerhalb angeordnet sein.

### IV. Beispiele für die Erfindungsausführung

Das Behandlungsverfahren und die Vorrichtung sind in den Zeichnungen dargestellt.

In Figur 1 ist das Schema der Vorrichtung mit Diaphragma im Gehäuse dargestellt.

In Figur 2 ist das Schema der Vorrichtung mit Diaphragma außerhalb des Vorrichtungsgehäuses dargestellt.

In Figur 3 ist das Schema der Vorrichtung mit Polarisator im Gehäuse dargestellt.

In Figur 4 ist das Schema der Vorrichtung mit Polarisator außerhalb des Gehäuses dargestellt.

Die Figuren 5-10 zeigen die Ansicht des Diaphragmas in isometrischer Projektion mit Maske in Form verschiedener geometrischer Figuren.

Die Vorrichtung zur Behandlung des Keratokonus besteht aus dem Gehäuse 1, in dem sich die UV-Lichtquelle 2 mit einer Wellenlänge von 350-380 nm und Elemente der optischen Anordnung 4 aus Quarzglas befinden. Die UV-Lichtquelle ist mit dem Netz- und Bedienungsgerät 3 verbunden, das die Stromversorgung sichert und Zeitdauer und Leistung der Vorrichtung reguliert. Die Vorrichtung enthält ebenfalls das Diaphragma 5, das als Maske mit einander abwechselnden lichtdurchlässigen und lichtundurchlässigen Abschnitten ausgeführt ist, die die Form von konzentrischen Kreisen, eines Gitters, von Kästchen, Spiralen, Bögen, parallelen Linien oder anderen geometrischen Figuren hat und auf einer optischen Achse mit den Elementen der optischen Anordnung 4 und der UV-Lichtquelle angeordnet ist. Dabei kann das Diaphragma 5 im Gehäuse oder außerhalb angeordnet werden. Bei Betrieb der Vorrichtung wird es auf einer optischen Achse mit der UV-Lichtquelle platziert. Die Behandlung geht folgendermaßen vonstatten.

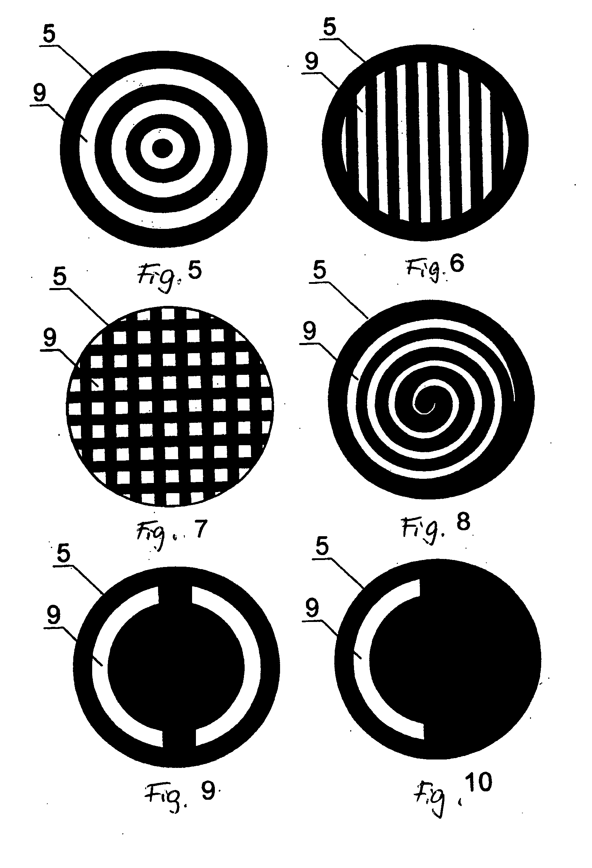

Nach vorhergehender Lokalanästhesie wird in das Auge der Photomediator geträufelt - 30 Minuten lang eine 1 %-ige Riboflavin-Lösung und mit gebündeltem UV-Licht durch das Diaphragma innerhalb von 1-90 Minuten bestrahlt. Durch die chemische Reaktion des Crosslinkings des Hornhautkollagens bilden sich darin stärker verfestigte Felder. Das Diaphragma ist in Form einer Maske mit einander abwechselnden lichtdurchlässigen und lichtundurchlässigen Abschnitten ausgeführt, die die Form von konzentrischen Kreisen, eines Gitters, von Kästchen, Spiralen, Bögen, parallelen Linien oder anderen geometrischen Figuren haben (Figuren 5-10), wodurch in der Hornhaut Bestrahlungsfelder entstehen können, die ein originelles Gebilde darstellen, wodurch die erforderliche Festigkeit der Hornhaut bei geringerer Einwirkungsfläche erreicht wird, was wiederum bei gleichbleibender Behandlungseffektivität die Wahrscheinlichkeit von Nebenwirkungen verringert bzw. mögliche Nebenwirkungen mildert.

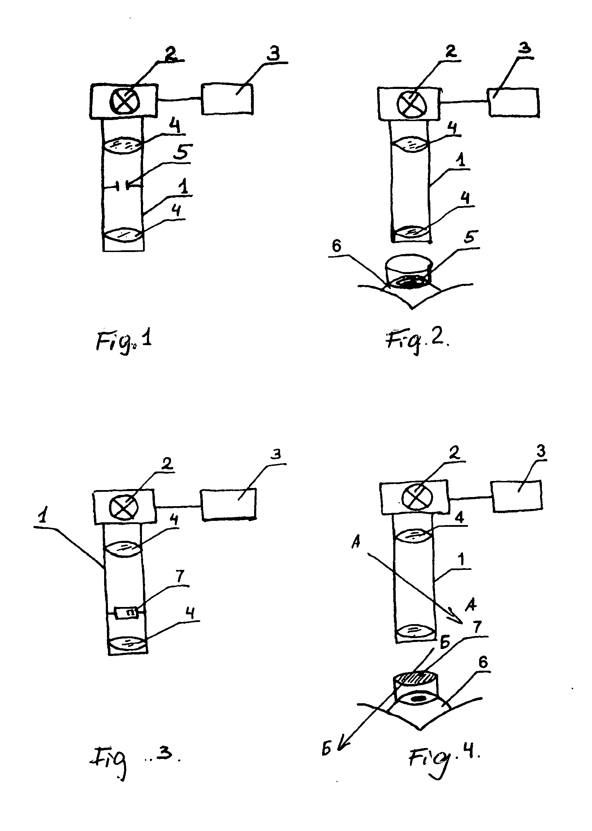

Eine weitere Variante der Vorrichtung beinhaltet das Gehäuse 1, in dem die UV-Lichtquelle 2 mit einer Wellenlänge von 350-380 nm und Elemente der optischen Anordnung 4 aus Quarzglas liegen. Die UV-Lichtquelle ist mit dem Netz- und Bedienungsgerät 3 verbunden, das die Stromversorgung sicherstellt und die Zeitdauer und die Betriebsleistung der UV-Lichtquelle reguliert. Die Vorrichtung schließt ebenfalls das Diaphragma 5 ein, das auf einer optischen Achse mit den Elementen der optischen Anordnung 4 und der UV-Lichtquelle angeordnet ist. Außerdem enthält die Vorrichtung den Polarisator 7, der auf der optischen Achse im Gehäuse oder außerhalb angeordnet ist. Der Polarisator kann in der Polarisationsebene um 360 Grad gedreht werden und das UV-Licht polarisieren. Durch Nutzung der Polarisationswirkung des Lichts durch die Hornhaut und durch Ausrichtung der Polarisationsebene des UV-Lichts zur Polarisationsebene der Hornhaut unter einem Winkel von 1 bis 180 Grad kann die Wirkungstiefe der UV-Strahlung reguliert werden, wobei die Wirkung der UV-Strahlung auf die gegenüber Schädigungen durch Licht empfindlichen endothelialen Zellen verringert wird, was wiederum bei gleichbleibender Behandlungseffektivität die Wahrscheinlichkeit von Nebenwirkungen verringert bzw. mögliche Nebenwirkungen mildert. In Figur 4 ist eine der Positionen der Polarisatorachse A-A zu B-B, der Polarisationsebene der Hornhaut des Auges, dargestellt. Die Behandlung geht folgendermaßen vonstatten.

Mittels eines Polarimeters wird die Polarisationsebene der Hornhaut bestimmt. Nach Lokalanästhesie wird 30 Minuten lang der Photomediator, z. B. eine 1%-ge Riboflavin-Lösung, in das Auge geträufelt, der Polarisator, dessen Polarisationsebene zur Polarisationsebene des Lichts durch die Hornhaut des Auges unter einem Winkel von 1 bis 180 Grad gedreht wird, auf einer optischen Achse mit der UV-Lichtquelle angebracht und die Hornhaut durch den Polarisator mit gebündeltem UV-Licht 1-90 Minuten bestrahlt. Durch die chemische Reaktion des Kollagencrosslinkings der Hornhaut bilden sich in ihr stärker verfestigte Felder. Die beschriebene Anordnung des Polarisators ermöglicht es, die Einwirkungstiefe des UV-Lichts zu regulieren, wobei die Wirkung des UV-Lichts auf die gegenüber einer Schädigung durch Licht empfindlichen endothelialen Zellen verringert wird, was wiederum bei gleichbleibender Behandlungseffektivität die Wahrscheinlichkeit von Nebenwirkungen verringert bzw. mögliche Nebenwirkungen mildert.

Nachstehend sind Beispiele für die Anwendung des Verfahrens angeführt.

### Beispiel 1

Patient A., 26 Jahre, Diagnose: Keratokonus beider Augen.

Visus vor der Operation:
Rechtes Auge - 0,01 sph - 4,0 D, cyl -4,0 D ax 85°
Linkes Auge - 0,2 sph - 1,5 D, cyl -1,5 D ax 95°

Im letzten halben Jahr war ein intensives Fortschreiten der Erkrankung zu beobachten.

Es wurde eine Operation nach dem erfindungsgemäßen Verfahren durchgeführt. Nach Lokalanästhesie wurde der Photomediator, eine 1%-ige Riboflavin-Lösung, 30 Minuten lang eingeträufelt. Dann wurde auf die Oberfläche der Hornhaut die Darstellung des Diaphragmas in Form von durch die UV-Lichtquelle gebildeten konzentrischen Kreisen projiziert. Die Expositionszeit betrug 1 Minute.

Einen Tag nach der Operation war das rechte Auge ruhig, zeigte keine Reaktion, kein Schmerzempfinden, bei der Untersuchung an der Spaltlampe keine Anzeichen einer Hornhautschädigung. Die Kontrolluntersuchung nach einem Jahr ergab, dass die Erkrankung im rechten Auge nicht fortschreitet. Nach dem gleichen Verfahren wurde das linke Auge behandelt.

### Beispiel 2

Patient B., 28 Jahre, Diagnose: Keratokonus beider Augen.

Visus vor der Operation:
Rechtes Auge - 0,01 sph - 2,0 D, cyl -5,0 D ax 65°
Linkes Auge - 0,1 sph- 1,5 D, cyl-3,5 D ax 105°

Im letzten halben Jahr war ein intensives Fortschreiten der Erkrankung zu beobachten.

Es wurde eine Operation nach dem erfindungsgemäßen Verfahren durchgeführt. Nach Lokalanästhesie wurde der Photomediator, eine 1%-ige Riboflavin-Lösung, 30 Minuten lang eingeträufelt, auf die Oberfläche der Hornhaut das Diaphragma in Form von durch die UV-Lichtquelle gebildeten parallelen Linien projiziert. Es wurde eine Ausrichtung der Linien parallel zur Astigmatismus-Achse gewählt, d. h. parallel zum Meridian von 65°. Das Diaphragma wurde mittels Vakuumring fixiert. Die Expositionszeit betrug 30 Minuten.

Einen Tag nach der Operation war das rechte Auge ruhig, zeigte keine Reaktion, kein Schmerzempfinden, bei der Untersuchung an der Spaltlampe keine Anzeichen einer Hornhautschädigung. Die Kontrolluntersuchung nach einem Jahr ergab, dass die Erkrankung im rechten Auge nicht fortschreitet. Nach dem gleichen Verfahren wurde das linke Auge behandelt.

### Beispiel 3:

Patient Sch., 38 Jahre, Diagnose: Keratokonus beider Augen.

Visus vor der Operation:
Rechtes Auge - 0,01 sph - 0,5 D, cyl -4,0 D ax 75°
Linkes Auge - 0,2 cyl -3,75 D ax 80°

Im letzten Jahr hat sich das Sehvermögen verschlechtert.

Es wurde eine Operation nach dem erfindungsgemäßen Verfahren durchgeführt. Mit Hilfe eines Polarimeters wurde die Polarisationsebene der Hornhaut bestimmt. Dann erfolgte die Lokalanästhesie. Nach dem Einträufeln des Photomediators, einer 1 %-igen Riboflavin-Lösung, 30 Minuten lang, wurde die Polarisationsebene des Polarisators der Vorrichtung unter einem Winkel von 90° zur Polarisationsebene der Hornhaut gestellt. Auf die Oberfläche der Hornhaut des rechten Auges wurde ein von der UV-Lichtquelle gebildeter Fleck projiziert. Die Expositionszeit betrug 40 Minuten.

Einen Tag nach der Operation war das rechte Auge ruhig, zeigte keine Reaktion, kein Schmerzempfinden, bei der Untersuchung an der Spaltlampe keine Anzeichen einer Hornhautschädigung. Die Kontrolluntersuchung nach einem Jahr ergab, dass die Erkrankung im rechten Auge nicht fortschreitet.

### Beispiel 4:

Patient A., 24 Jahre, Diagnose: Beidseitiger Keratokonus.

Visus vor der Operation:
Rechtes Auge - 0,05 sph - 0,25 D, cyl -4,5 D ax 65°
Linkes Auge - 0,2 cyl -3,5 D ax 105°

Im letzten Jahr hat sich das Sehvermögen verschlechtert.

Es wurde eine Operation nach dem erfindungsgemäßen Verfahren durchgeführt. Mit Hilfe eines Polarimeters wurde die Polarisationsebene der Hornhaut bestimmt. Dann erfolgte die Lokalanästhesie. Nach dem Einträufeln des Photomediators, der 1 %-igen Riboflavin-Lösung, 30 Minuten lang, wurde die Polarisationsebene des Polarisators der Vorrichtung unter einem Winkel von 90° zur Polarisationsebene der Hornhaut gestellt. Auf die Oberfläche der Hornhaut des rechten Auges wurde ein von der UV-Lichtquelle gebildeter Fleck projiziert. Die Expositionszeit betrug 90 Minuten.

Einen Tag nach der Operation war das rechte Auge ruhig, zeigte keine Reaktion, kein Schmerzempfinden, bei der Untersuchung an der Spaltlampe keine Anzeichen einer Hornhautschädigung. Die Kontrolluntersuchung nach einem Jahr ergab, dass die Erkrankung im rechten Auge nicht fortschreitet.

## Patentansprüche

1. Verfahren zur Behandlung des Keratokonus mit UV-Strahlen, das in der Lokalanästhesie, der Einträufelung des Photomediators, der UV-Bestrahlung der Hornhaut des Auges innerhalb von 1-90 Minuten besteht, **dadurch gekennzeichnet, dass** eine bzw. mehrere UV-Bestrahlungsfelder unterschiedlicher Form gebildet werden, u. a. in Form von konzentrischen Kreisen, Bögen, parallelen Linien, Kästchen, eines Gitters, von Spiralen oder anderen geometrischen Figuren.

2. Verfahren zur Behandlung des Keratokonus mit UV-Strahlen, das in der Lokalanästhesie, der Einträufelung des Photomediators, der UV-Bestrahlung der Hornhaut des Auges innerhalb von 1-90 Minuten besteht, **dadurch gekennzeichnet, dass** das UV-Licht polarisiert und die Polarisationsebene zur Polarisationsebene der Hornhaut unter einem Winkel von 1 bis 180 Grad ausgerichtet wird.

3. Vorrichtung zur Behandlung des Keratokonus mit UV-Strahlen, die ein Gehäuse mit darin befindlicher UV-Lichtquelle, ein damit verbundenes Netz- und Bedienungsgerät, Fokussierelemente, die auf einer optischen Achse mit der UV-Lichtquelle liegen, und ein Diaphragma einschließt, **dadurch gekennzeichnet, dass** das Diaphragma in Form einer Maske mit einander abwechselnden lichtdurchlässigen und lichtundurchlässigen Abschnitten, die die Form von konzentrischen Kreisen, eines Gitters, von Kästchen, Bögen, parallelen Linien bzw. anderen geometrischen Figuren haben, ausgeführt ist, das Diaphragma auf einer optischen Achse mit der UV-Lichtquelle angeordnet werden soll.

4. Vorrichtung nach Anspruch 3, **dadurch gekennzeichnet, dass** das Diaphragma im Vorrichtungsgehäuse angeordnet ist.

5. Vorrichtung nach Anspruch 3, **dadurch gekennzeichnet, dass** das Diaphragma außerhalb des Vorrichtungsgehäuses und auf der Hornhaut bzw. zwischen dem Vorrichtungsgehäuse und der Hornhaut liegt.

6. Vorrichtung zur Behandlung des Keratokonus mit UV-Strahlen, die ein Gehäuse mit darin befindlicher UV-Lichtquelle, ein damit verbundenes Netz- und Bedienungsgerät, Fokussierelemente, die auf einer optischen Achse mit der UV-Lichtquelle liegen, und ein Diaphragma einschließt, **dadurch gekennzeichnet, dass** sie einen Polarisator enthält, der auf einer optischen Achse mit der UV-Lichtquelle angeordnet werden soll und so ausgeführt ist, dass die Polarisationsebene des UV-Lichts zur Polarisationsebene der Hornhaut in einem Winkel von 1 bis 180 Grad ausgerichtet werden kann.

7. Vorrichtung nach Anspruch 6, **dadurch gekennzeichnet, dass** der Polarisator im Vorrichtungsgehäuse angeordnet ist.

8. Vorrichtung nach Anspruch 6, **dadurch gekennzeichnet, dass** der Polarisator außerhalb des Vorrichtungsgehäuses angeordnet ist und auf der Hornhaut bzw. zwischen Vorrichtungsgehäuse und Hornhaut liegt.
